# EUROPÄISCHE PATENTSCHRIFT

(11) **EP 0 966 918 B1**
(45) Veröffentlichungstag und Bekanntmachung des Hinweises auf die Patenterteilung: **12.10.2005**
(21) Anmeldenummer: 99111589.0
(22) Anmeldetag: 15.06.1999
(51) Int. Cl.: A61B 6/00, G01T 1/29

(54) **Mammographie-Röntgengerät mit einem Festkörper-Detektor**
X-ray mammograph provided with a solid state detector
Mammographe à rayons X pourvu d'un détecteur à l'état solide

(30) Priorität: 23.06.1998 DE 19827964
(43) Veröffentlichungstag der Anmeldung: 29.12.1999
(73) Patentinhaber: SIEMENS AKTIENGESELLSCHAFT, 80333 München (DE)
(72) Erfinder: Hoheisel, Martin Dr., 91056 Erlangen (DE); Kirsch, Jürgen, 85667 Oberpframmern (DE); Sklebitz, Hartmut Dipl.-Ing.(FH), 91056 Erlangen (DE); Spahn, Martin Dr., 91054 Erlangen (DE)

(56) Entgegenhaltungen:
- EP-A- 0 714 038
- EP-A- 0 776 126
- WO-A-96/35372
- DE-A- 4 402 114
- DE-C- 19 705 755
- US-A- 5 454 022
- US-A- 5 528 043

## Beschreibung

Die Erfindung betrifft ein Mammographie-Röntgengerät mit einer Röntgenröhren-Anordnung, einer Kompressionsvorrichtung, einem Objekttisch und einem Festkörper-Detektor. Derartige Röntgendiagnostikeinrichtungen dienen zur Untersuchung der Mamma einer Patientin.

Heutige weit verbreitete Mammographie-Röntgengeräte arbeiten mit Film-Folien-Systemen. Diese Systeme benötigen keinen großen Raumbedarf, da der Bildsensor keine elektrischen Signale zum Gerät übermitteln muß.

Aus dem Siemens Datablatt "MAMMOMAT 3000, OPDIMA - Digital biopsy and spot imaging system", Druckzeichen WS 0597 7, ist ein mammographisches Röntgengerät bekannt, bei dem in einer Kassette ein CCD-Bildsensor angeordnet ist, der eine in Bezug auf das Kassettenformat von 18*24 cm² kleine Bildfläche von 49*85 mm² aufweist. Diese CCD-Kassette ist über ein Kabel mit einer Workstation verbunden, die eine Wiedergabe der mammographischen Röntgenbilder bewirkt. Da der CCD-Bildsensor von der Kassette nur wenig Raum in Anspruch nimmt, kann die nötige Elektronik problemlos in dieser Kassette untergebracht werden. Als nachteilig wirkt sich die sehr kleine Detektorfläche aus, die keine aus der Filmtechnik bekannte großformatige Untersuchung erlaubt. Sollen größere Bereiche untersucht werden, so sind mehrere Untersuchungen notwendig, die eine entsprechend erhöhte Strahlenbelastung der Untersuchungsperson bedeuten. Weiterhin wirkt sich das Kabel als störend aus.

Bei neueren Mammographie-Röntgengeräten werden Festkörper-Bildsensoren beispielsweise a-Si Panels eingesetzt, bei denen jedoch umfangreiche und z.T. voluminöse Ansteuer- und Ausleseelektroniken am Detektor angeordnet sind, so daß derartige Detektoren ein großes Volumen aufweisen. Diese dürfen jedoch nicht die Vielfalt der diagnostischen Methoden der Bildaufnahme einschränken, um die Akzeptanz derartiger Geräte nicht einzuschränken, so daß an Detektorfläche gespart werden muß, um die Abmessungen klein zu halten.

Weiterhin gibt es relativ dicke Sensor-Arrays mit optischen Tapern, die die Anwendung bei adipösen Patientinnen erschwert oder unmöglich macht.

Die Ausmaße herkömmlicher Festkörper-Detektoren überschreiten in Länge und Breite die eigentlich strahlungsempfindliche Fläche oft in erheblichem Umfang. Für einen Festkörper-Mammographie-Detektor ist dies nicht akzeptabel. Deshalb müssen spezielle Maßnahmen getroffen werden, die es ermöglichen, die aktive Fläche millimetergenau bis an den Rand der Kassette auszudehnen.

Die Erfindung geht von der Aufgabe aus, ein Mammographie-Gerät der eingangs genannten Art zu schaffen, das wechselweise eine Aufnahme mit einem Film und einem Festkörperbildwandler ermöglicht und derart kompakt gebaut ist, daß ein freier und nahezu ungehinderter Zugang zu der zu untersuchenden Patientin möglich ist.

Die Aufgabe wird erfindungsgemäß dadurch gelöst, daß ein großflächiger Festkörper-Detektor aus amorphem Silizium (aSi) in einer in den Objekttisch einschiebbaren Detektor-Kassette integriert ist, die einen ersten Teil wenigstens einer Übertragungsvorrichtung für die Zufuhr der Betriebsspannung, der Steuersignale zum Betreiben des Festkörper-Detektors und/oder der Auslesedaten aufweist, der in ein entsprechenden, an dem Objekttisch angebrachten zweiten Teil der Übertragungsvorrichtung wirkungsmäßig eingreift.

Es hat sich als vorteilhaft erwiesen, wenn der Festkörper-Detektor einteilig ausgebildet ist.

In vorteilhafter weise kann als Übertragungsvorrichtung für die Zufuhr der Betriebsspannung der Objekttisch und die Detektor-Kassette mit mindestens zwei elektrischen, lösbaren Verbindungen, mit einem induktiven Übertrager oder mit einem kapazitiven Übertrager für die Betriebsspannung versehen sein.

In vorteilhafter Weise kann der Objekttisch und die Detektor-Kassette einen optischen, kapazitiven oder induktiven Übertrager für die Steuersignale zum Betreiben des Panels aufweisen.

Um bei einem Mammographie-Röntgengerät mit einem Festkörper-Detektor, der eine Dunkel-Referenz-Zone (DRZ) zur Verringerung des Zeilenrauschens aufweist, die aktive Fläche millimetergenau bis an den Rand der Kassette auszudehnen hat es sich als vorteilhaft erwiesen, wenn die Dunkel-Referenz-Zone auf der Geräteseite des Festkörper-Detektors angeordnet ist.

Um den Platz in der Detektor-Kassette möglichst für den Festkörper-Detektor verwenden zu können, so daß die aktive Fläche des Festkörper-Detektors nahezu die gleichen Abmessungen wie die Fläche der Kassette aufweist, kann eine Detektor-Elektronik in einem Bereich angeordnet sein, der sich zwischen dem Dreharm des Röntgengerätes und der Detektor-Kassette mit dem Festkörper-Detektor befindet.

Bei einem Mammographie-Röntgengerät, bei dem eine die Detektor-Elektronik tragende Platine mit dem Festkörper-Detektor über wenigstens ein flexibles Leiterband verbunden ist, hat es sich als zweckmäßig erwiesen, wenn wenigstens ein Ende der Leiterbänder mit einem vorgeformten Knick versehen ist, der durch Prägung der Leiterbänder erzeugt sein kann. Dadurch läßt sich die Breite des Gehäuses der Detektor-Kassette derart reduzieren, daß die aktive Fläche bis an den Rand der Kassette reicht, da ein raumzehrender Bogen nicht benötigt wird.

Die Erfindung ist nachfolgend anhand von in der Zeichnung dargestellten Ausführungsbeispielen näher erläutert. Es zeigen:
- Figur 1: ein Mammographie-Gerät zum Einsatz einer erfindungsgemäßen Vorrichtung mit Festkörper-Detektor,
- Figur 2: eine erfindungsgemäße Detektor-Kassette zum Einsatz in einem Mammographie-Gerät gemäß Figur 1,
- Figur 3: einen Teil eines erfindungsgemäßen Festkörper-Detektors gemäß Figur 2 und
- Figur 4: einen schematischen Querschnitt durch einen Festkörper-Detektor.

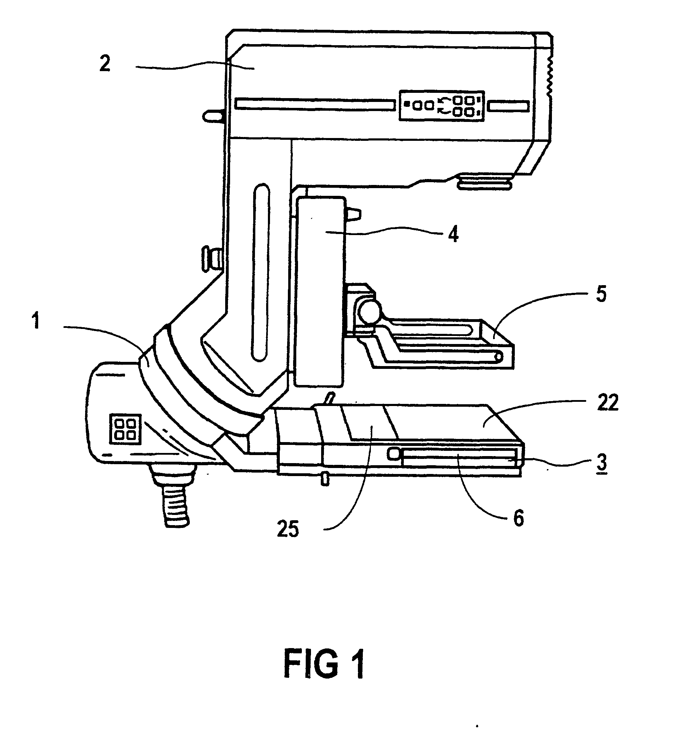

In der Figur 1 ist ein mammographisches Röntgengerät mit einem Dreharm 1 dargestellt, der eine Röntgenröhren-Anordnung 2 mit Kollimator, einen Objekttisch 3 und eine Kompressionsvorrichtung 4 mit Kompressionsplatte 5 aufweist. In den Objekttisch 3 läßt sich seitlich eine Detektor-Kassette 6 mit einem großflächigen Festkörper-Detektor 7 einschieben, der erfindungsgemäß ein aSi-Halbleiterdetektor ist. Dieser weist eine bildgebende Detektorfläche 22 auf.

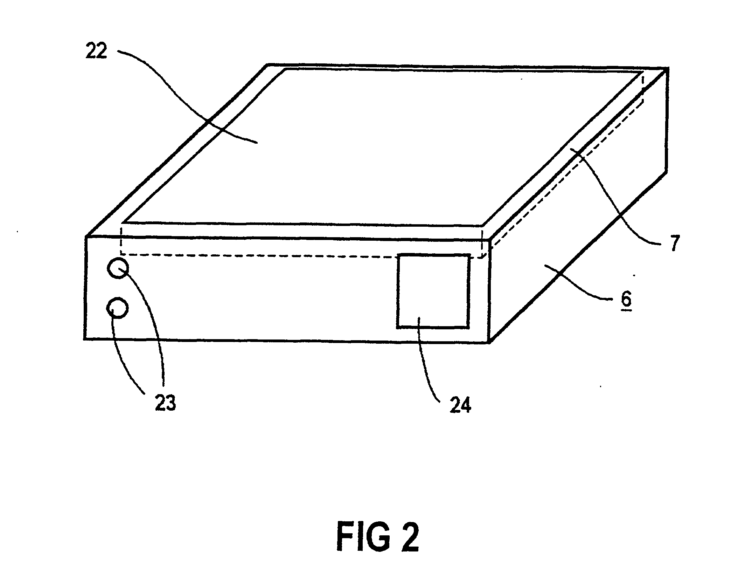

Die anhand von Figur 2 dargestellte Detektor-Kassette 6 mit dem Festkörper-Detektor 7 ist mit elektrischen Kontakten 23 für die Stromversorgung des Festkörper-Detektors und einem Übertrager 24 für die Steuersignale zum Betreiben des Festkörper-Detektors als kassettenseitiger Teil von Übertragungsvorrichtungen versehen, die in entsprechende, an dem Objekttisch 3 angebrachte Teile wirkungsmäßig eingreifen. Die Signale für die Aufnahme-Auslösung, eventuelle Belichtungs Modi, Takte zum Start der spaltenweisen Auslesung, Takte zum zeilenweisen Auslesen, Taktsignale zum Rücksetzen des Panels nach dem Auslesen bzw. zum Start eines Belichtungsvorgangs beim Röntgenschuß können optisch, kapazitiv oder induktiv auf diese Detektor-Kassette 6 übertragen werden. Die Zuführung der Betriebsspannung(en) kann auch induktiv oder kapazitiv über weitere nicht dargestellte Übertrager erfolgen.

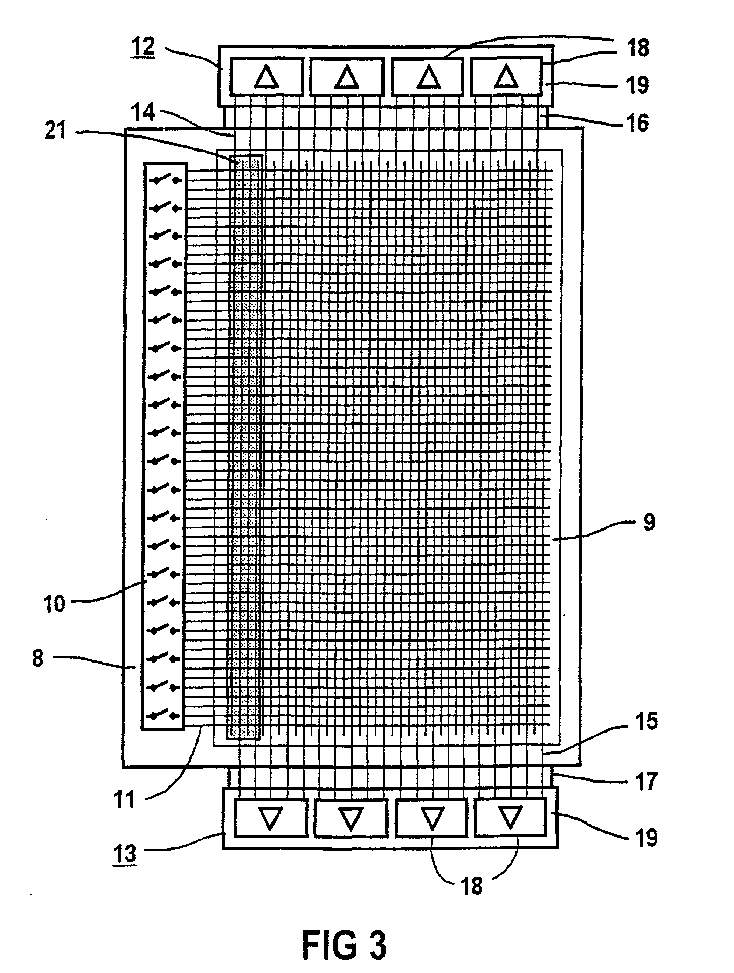

In Figur 3 ist dieser Festkörper-Detektor dargestellt, der ein auf einem Glassubstrat 8 angeordnetes matrixförmiges Array 9, die aktive aSi Photodioden-Matrix, aufweist, die aus einzelnen lichtempfindlichen Zellen besteht, die in n-Zeilen und m-Spalten angeordnet sind. Diese Zellen, denen ein Röntgenkonverter vorgeordnet ist, sind mit einer Ansteuerelektronik 10 über Zeilenleitungen 11 und mit beidseitig des Glassubstrat 8 angeordneten Ausleseelektroniken 12 und 13 über Spaltenleitungen 14 und 15 verbunden. Die Anschlüsse der Ausleseelektronik 12 und 13 werden beidseitig dem Array 9 zugeführt, wobei die Spaltenleitungen 14 und 15 derart verschachtelt ausgeführt sind, daß die Spalten alternierend mal nach oben von der Ausleseelektronik 12 und mal nach unten von der Ausleseelektronik 13 ausgelesen werden. Sie können aber auch derart angeordnet sein, daß die Spaltenleitungen 14 und 15 zur Reduzierung von parasitären Kapazitäten in der Mitte unterbrochen sind, so daß die Ausleseelektronik 12 den gesamten oberen Bereich und die Ausleseelektronik 13 den gesamten unteren Bereich der Pixel erfaßt.

Die einzelnen lichtempfindlichen Zellen der aktiven aSi Matrix können beispielsweise in bekannter Weise jeweils aus einer Photodiode und einer Schaltdiode bestehen, deren Kathoden miteinander verbunden sind und deren Anoden an den Zeilen- 11 bzw. Spaltenleitungen 14 und 15 angeschlossen sind. Es lassen sich aber auch andere Schaltelemente, beispielsweise TFTs verwenden.

Die Ansteuerelektronik 10 kann aus polykristallinem Silicium auf dem Glassubstrat 8 aufgebracht sein, während die Ausleseelektronik 12 und 13 diskret mit externen elektronischen Bausteinen 18, beispielsweise integrierten Schaltungen (ICs oder ASICs), aufgebaut sein kann. Die elektronischen Bausteine 18, die Auslesechips, können Vorverstärkerstufen und Multiplexer enthalten.

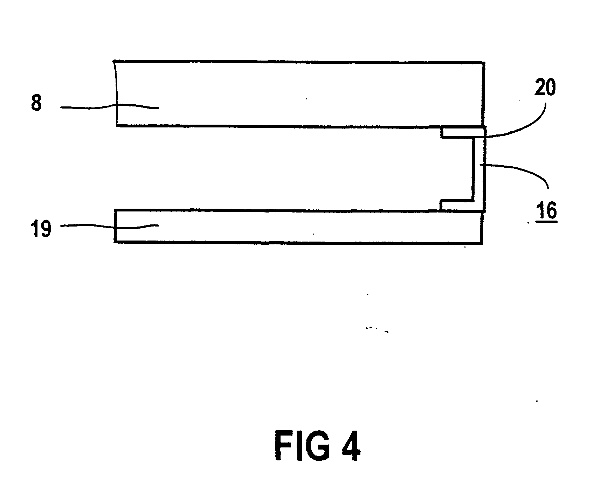

Über Verbindungen beispielsweise durch flexible Leiterbänder 16 und 17 sind die elektronischen Bausteine 18 mit dem aktiven aSi-Array 9 verbunden. Die elektronischen Bausteine 18 können dabei auf einer oder mehrerer in Figur 4 dargestellten unterhalb des Glassubstrats 8 angeordneten Platinen 19 angebracht sein. Die flexiblen Leiterbänder 16 und 17 sind erfindungsgemäß mit beispielsweise durch Prägung vorgeformte Knikke 20 versehen, so daß sie seitlich nicht mehr oder nur geringfügig überstehen müssen. Durch diese erfindungsgemäßen flexiblen Leiterbänder 16 und 17 ist es möglich, nahezu den gesamten Bereich der für die Bildgebung vorgesehenen Fläche sowohl seitlich als auch in Richtung auf die Untersuchungsperson zu verwenden.

Die elektronischen Bausteine 18 sind über nicht dargestellte Verbindungsleitungen mit den weiteren Bausteinen der Ausleseelektronik 12 und 13 verbunden, die Verstärkerstufen und Analog/Digital-Wandler (A/D-Wandler) aufweisen kann. Diese weiteren Baustein können auf der gleichen Platine 19 oder einer weiteren Elektronikplatine angeordnet sein. Erfindungsgemäß sind diese in einem Bereich 25 seitlich neben der Detektor-Kassette 6 mit dem Festkörper-Detektor 7 in Richtung zum Dreharm 1 des Röntgengerätes angeordnet. Aber auch die Ausleseelektronik 12 und 13 kann in diesem Bereich 25 angeordnet sein.

In der DE 195 27 148 C1 ist ein Verfahren zur Defekterkennung bei einem a-Si-Panel beschrieben, bei dem zur Erkennung von defekten Bildpunkten auch ein Signal einer sogenannten Dunkel-Referenz-Zone (DRZ) Verwendung findet. Diese Dunkel-Referenz-Zone liegt am linken Bildrand, dem Zeilenanfang beim Radiographie-Festkörper-Detektor, beispielsweise einem aSi-Detektor. Sie ist licht- und strahlungsdicht abgedeckt, so daß auch im Hellbild kein zusätzliches Signal entsteht und kann bis zu einigen hundert Spalten umfassen. Die Signalwerte der unbelichteten Pixel werden zur Korrektur des Zeilensignals herangezogen. Sie dienen dazu, das Zeilenrauschen zu vermindern und kleine Transienten im Offset zu korrigieren. Erfindungsgemäß ist nun diese Dunkel-Referenz-Zone 21 auf der der Brustwand gegenüberliegenden Seite des Panels in Richtung auf den Dreharm 1 des Röntgengerätes angeordnet, so daß sich keine Einschränkung des Bildfeldes an der Brustwand ergibt.

Erfindungsgemäß werden bei einem bekannten a-Si-Panel mehrere Verbesserungen durchgeführt, die den platzsparenden Einsatz eines Festkörper-Detektors in einem bekannten Mammographie-Röntgengerät ermöglichen:
- Das a-Si-Panel wird in einer Röntgen-Kassette integriert.
- Diese Kassette weist beispielsweise mindestens 2 elektrische Kontakte zur Zuführung der Betriebsspannung(en) auf.
- Die Ansteuersignale zum Betreiben des Panels wie Aufnahme-Auslösung, Belichtungs Modi, Taktsignale zum Start der spaltenweisen Auslesung, zum zeilenweisen Auslesen und zum Rücksetzen des Panels nach dem Auslesen bzw. zum Start eines Belichtungsvorgangs beim Röntgenschuß werden optisch, kapazitiv oder induktiv auf diese Kassette übertragen.
- Die Dunkel-Referenz-Zone DRZ wird auf der der Brustwand gegenüberliegenden Seite des Panels angeordnet.
- Um die diagnostische Bildgebung beispielsweise nahe der Achsel nicht zu Beschränken, werden ein oder beide Enden eines Leiterbandes mittels vorgeformtem Knick im ausgestattet.
- Um die diagnostischen Methoden nicht einzuschränken, indem der Volumen-Bedarf nahe am Patienten gering gehalten und insbesondere eine geringe Dicke der Sensor-Anordnung erreicht wird, wird die nicht direkt am Panel erforderliche Detektor-Elektronik im Bereich 25 zwischen Festkörper-Detektor 7 und Dreharm 1 des Röntgengerätes angeordnet.

## Patentansprüche

1. Mammographie-Röntgengerät mit einer Röntgenröhren-Anordnung (2), einer Kompressionsvorrichtung (4), einem Objekttisch (3) und einem großflächigen Festkörper-Detektor (7) aus amorphem Silizium (aSi), der in einer in den Objekttisch (3) einschiebbaren Detektor-Kassette (6) integriert ist, die einen ersten Teil wenigstens einer Übertragungsvorrichtung (23, 24) für die Zufuhr der Betriebsspannung, der Steuersignale zum Betreiben des Festkörper-Detektor (7) und/oder der Auslesedaten aufweist, der in einen entsprechenden, an dem Objekttisch (3) angebrachten zweiten Teil der Übertragungsvorrichtungen (23, 24) wirkungsmäßig eingreift.

2. Mammographie-Röntgengerät nach Anspruch 1, **dadurch gekennzeichnet, daß** der Festkörper-Detektor (7) mit einem einteiligen Substrat ausgebildet ist.

3. Mammographie-Röntgengerät nach Anspruch 1 oder 2, **dadurch gekennzeichnet, daß** der Objekttisch (3) und die Detektor-Kassette (6) mit mindestens zwei elektrischen, lösbaren Verbindungen (23) zur Zuführung der Betriebsspannung versehen sind.

4. Mammographie-Röntgengerät nach Anspruch 1 oder 2, **dadurch gekennzeichnet, daß** der Objekttisch (3) und die Detektor-Kassette (6) mit einem induktiven Übertrager für die Betriebsspannung versehen sind.

5. Mammographie-Röntgengerät nach Anspruch 1 oder 2, **dadurch gekennzeichnet, daß** der Objekttisch (3) und die Detektor-Kassette (6) mit einem kapazitiven Übertrager für die Betriebsspannung versehen sind.

6. Mammographie-Röntgengerät nach einem der Ansprüche 1 bis 5, **dadurch gekennzeichnet, daß** der Objekttisch (3) und die Detektor-Kassette (6) einen Übertrager (24) für die Steuersignale zum Betreiben des Festkörper-Detektors (7) aufweist.

7. Mammographie-Röntgengerät nach Anspruch 6, **dadurch gekennzeichnet, daß** als Übertrager (24) für die Steuersignale ein optischer Übertrager vorgesehen ist.

8. Mammographie-Röntgengerät nach Anspruch 6, **dadurch gekennzeichnet, daß** als Übertrager (24) für die Steuersignale ein kapazitiver Übertrager vorgesehen ist.

9. Mammographie-Röntgengerät nach Anspruch 6, **dadurch gekennzeichnet, daß** als Übertrager (24) für die Steuersignale ein induktiver Übertrager vorgesehen ist.

10. Mammographie-Röntgengerät mit einem Festkörper-Detektor (7), der eine Dunkel-Referenz-Zone (DRZ 21) zur Verringerung des Zeilenrauschens aufweist, nach einem der Ansprüche 1 bis 9, **dadurch gekennzeichnet, daß** die Dunkel-Referenz-Zone (21) auf der Geräteseite des Festkörper-Detektors (7) angeordnet ist.

11. Mammographie-Röntgengerät nach einem der Ansprüche 1 bis 10, **dadurch gekennzeichnet, daß** eine Detektor-Elektronik (12,13) in einem Bereich (25) angeordnet ist, der sich zwischen dem Dreharm (1) des Röntgengerätes und der Detektor-Kassette (6) mit dem Festkörper-Detektor (7) befindet.

12. Mammographie-Röntgengerät nach einem der Ansprüche 1 bis 11, bei dem eine die Detektor-Elektronik (12, 13) tragende Platine mit dem Festkörper-Detektor (7) über wenigstens ein flexibles Leiterband verbunden ist, **dadurch gekennzeichnet, daß** wenigstens ein Ende des Leiterbandes (16, 17) mit einem vorgeformten Knick (20) versehen ist.

13. Mammographie-Röntgengerät nach Anspruch 12, **dadurch gekennzeichnet, daß** der vorgeformte Knick (20) durch Prägung der Leiterbahnen erzeugt ist.

## Claims

1. X-ray mammography device with an x-ray tube arrangement (2), a compression device (4), a subject table (3) and a large-area solid state detector (7) made from amorphous silicon (aSi), which is integrated into a detector cassette (6) which can be inserted into the subject table (3), which comprises a first part of at least one transmission device (23, 24) for the supply of operating voltage, a transmission path for supplying control signals to operate said solid state detector (7) and/or a transmission path for reading-out data, said subject table having a corresponding second part of the transmission device (23,24) inserted into the subject table (3) in an effective manner.

2. X-ray mammography device according to claim 1, **characterised in that**, the solid state detector (7) is designed with a one-piece substrate

3. X-ray mammography device according to claim 1 or 2, **characterised in that**, the subject table (3) and the detector cassette (6) are provided with at least two electrical, unlockable connections (23) for supplying the operating voltage.

4. X-ray mammography device according to claim 1 or 2, **characterised in that**, the subject table (3) and the detector cassette (6) are provided with an inductive transmitter for the operating voltage.

5. X-ray mammography device according to claim 1 or 2, **characterised in that**, the subject table (3) and the detector cassette (6) are provided with a capacitive transmitter for the operating voltage.

6. X-ray mammography device according to one of claims 1 to 5, **characterised in that**, the subject table (3) and the detector cassette (6) comprise a transmitter (24) for the control signals for operating the solid state detector (7).

7. X-ray mammography device according to claim 6, **characterised in that**, an optical transmitter is provided as a transmitter (24) for the control signals.

8. X-ray mammography device according to claim 6, **characterised in that**, a capacitive transmitter is provided as a transmitter (24) for the control signals.

9. X-ray mammography device according to claim 6, **characterised in that**, an inductive transmitter is provided as a transmitter (24) for the control signals.

10. X-ray mammography device with a solid state detector (7), having a dark reference zone (DNZ 21) to reduce the quantum noise according to one of claims 1 to 9, **characterised in that** the dark reference zone (21) is arranged on the device side of the solid state detector (7).

11. X-ray mammography device according to one of claims 1 to 10, **characterised in that** a detector electronics (12,13) is arranged in an area (25) located between the rotating arm (1) of the x-ray device and the detector cassette (6) with the solid state detector (7).

12. X-ray mammography device according to one of claims 1 to 11, in which one of the circuit boards bearing the detector electronics (12, 13) is connected to the solid state detector (7) by means of at least one flexible strip conductor **characterised in that** at least one end of the strip conductor (16, 17) is provided with a deformed break (20).

13. X-ray mammography device according to claim 12, **characterised in that**, the deformed break (20) is generated by stamping the conductor paths.

## Revendications

1. Mammographe à rayons X comportant un système de tubes à rayons X (2), un dispositif de compression (4), un plateau porte-objets (3) et un détecteur à semi-conducteurs (7) de grande surface en silicium amorphe (aSi) intégré dans une cassette de détection (6) qui peut être insérée dans le plateau porte-objets (3) et qui présente une première partie d'au moins un dispositif de transmission (23, 24) destiné à acheminer la tension de service, les signaux de commande qui font fonctionner le détecteur à semi-conducteurs (7) et/ou les données de lecture, cette première partie pénétrant activement dans une deuxième partie correspondante - appliquée au plateau porte-objets (3) - du dispositif de transmission (23, 24).

2. Mammographe à rayons X selon la revendication 1, **caractérisé en ce que** le détecteur à semi-conducteurs (7) est formé par un substrat en une pièce.

3. Mammographe à rayons X selon la revendication 1 ou 2, **caractérisé en ce que** le plateau porte-objets (3) et la cassette de détection (6) sont munis d'au moins deux liaisons (23) électriques amovibles pour acheminer la tension de service.

4. Mammographe à rayons X selon la revendication 1 ou 2, **caractérisé en ce que** le plateau porte-objets (3) et la cassette de détection (6) sont munis d'un transmetteur inductif pour la tension de service.

5. Mammographe à rayons X selon la revendication 1 ou 2, **caractérisé en ce que** le plateau porte-objets (3) et la cassette de détection (6) sont munis d'un transmetteur capacitif pour la tension de service.

6. Mammographe à rayons X selon l'une des revendications 1 à 5, **caractérisé en ce que** le plateau porte-objets (3) et la cassette de détection (6) présentent un transmetteur (24) destiné aux signaux de commande pour faire fonctionner le détecteur à semi-conducteurs (7).

7. Mammographe à rayons X selon la revendication 6, **caractérisé en ce que** le transmetteur (24) prévu pour les signaux de commande est un transmetteur optique.

8. Mammographe à rayons X selon la revendication 6, **caractérisé en ce que** le transmetteur (24) prévu pour les signaux de commande est un transmetteur capacitif.

9. Mammographe à rayons X selon la revendication 6, **caractérisé en ce que** le transmetteur (24) prévu pour les signaux de commande est un transmetteur inductif.

10. Mammographe à rayons X comportant un détecteur à semi-conducteurs (7) muni d'une zone de référence sombre (DRZ 21) pour réduire le bruit de rangée selon l'une des revendications 1 à 9, **caractérisé en ce que** la zone de référence sombre (21) est placée sur le côté du détecteur à semi-conducteurs (7) tourné vers l'appareil.

11. Mammographe à rayons X selon l'une des revendications 1 à 10, **caractérisé en ce qu'**une électronique de détection (12, 13) est placée dans une zone (25) située entre le bras tournant (1) de l'appareil radiographique et la cassette de détection (6) équipée du détecteur à semi-conducteurs (7).

12. Mammographe à rayons X selon l'une des revendications 1 à 11 dans lequel une platine qui supporte l'électronique de détection (12, 13) est reliée au détecteur à semi-conducteurs (7) par au moins une bande conductrice flexible, **caractérisé en ce qu'**au moins une extrémité de la bande conductrice (16, 17) est munie d'un coude (20) préformé.

13. Mammographe à rayons X selon la revendication 12, **caractérisé en ce que** le coude (20) préformé est produit en estampant les bandes conductrices.
